# EUROPEAN PATENT APPLICATION

(11) **EP 3 763 365 A1**
(43) Date of publication of application: **13.01.2021**
(21) Application number: 19185350.6
(22) Date of filing: 09.07.2019
(51) Int. Cl.: A61K 31/4166, A61K 9/00, A61P 37/08, A61Q 17/02, A61Q 17/04

(54) **AROYLIMIDAZOLONES FOR THE TREATMENT OF THAUMETOPOEA EXPOSURE**

(71) Applicant: Breakthrough Medical Research B.V., 1355 HK Almere (NL)
(72) Inventor: BEUTE, Jan, 1355 HA Almere (NL)
(74) Representative: Nederlandsch Octrooibureau

(57) **Abstract**

The invention involves the use of aroylimidazolones such as enoximone in the treatment of conditions caused by exposure to the burning hairs of a caterpillar of the genus *Thaumetopoea,* such as an oak processionary caterpillar. The invention also relates to compositions for use in such treatment, and to a method of treatment of a condition resulting from exposure to urticating hairs of a caterpillar of the genus *Thaumetopoea,* wherein the method comprises administering an aroylimidazolone or a pharmaceutically acceptable salt thereof to a subject exposed to said urticating hairs.

## Description

The *Thaumetopoea* moths are a family of moths whose larvae, or caterpillars, displace themselves in columns in search of food, resembling a procession. Besides damaging nature with their phyto- and xylophagous behaviour, these processionary caterpillars constitute a health hazard. The hairs of the caterpillar cause dermatological reactions in both animals and humans. Although the results, such as dermatitis, occurs among outdoor professionals, it is primarily extraprofessional.

Contamination generally occurs in woods or wooded areas, rarely in cities. Means of contamination comprise direct contact with the nest or the processional caterpillar and indirect contact with air dispersed hairs. The dermatitis is generally observed in late spring and particularly from April to June, among campers, tourers, hikers, or their companion animals. Pathologic eruption has its onset 1-12 hours after contact with the hairs and often presents with intense and continuous itching. Morphologically, it can be strophulus-like and consist of papulous, excoriated, and pinkish lesions on an oedematous base. Diagnosis is usually straightforward (Bonamonte et al., Sci. World J., 2013, Article ID 867431; DOI: 10.1155/2013/867431). The effect is caused by the urticating setae, or itching hairs, carried by the processionary caterpillars. These urticating hairs cause the health problems in humans and other warm-blooded animals.

It is worrying that several processionary caterpillars, such as the pine processionary moth *Thaumetopoea pityocampa* and the oak processionary moth *Thaumetopoea processionea* have responded to global warming (climate change) by extending their distribution ranges. The subfamily *Thaumetopoeinae* consists of approximately 100 species. It is unknown how the health hazard associated with these caterpillars can be contained. Another important question is whether other processionary moth species will similarly respond to these specific dimensions of global change and thus introduce additional health hazards, or introduce hazards into new areas.

The pathogenetic mechanism of the affection is mechanical, pharmacological, and allergic in nature. Besides skin, processionary caterpillars can affect the eyes and sometimes the airways. Despite the considerable damage to humans, animals, and nature, the medical literature has only few studies, supplemented by reports from local media and by folk wisdom. There is a need for medical treatment of the adverse effects that exposure to *Thaumetopoea* caterpillars can cause in subjects, both humans and pets.

This invention involves the use of aroylimidazolone to treat these adverse effects. Aroylimidazolones, particularly aroyl-2H-imidazol-2-on, or a pharmaceutically acceptable salt thereof, are widely known in the art. An exemplary aroylimidazolone is enoximone or a pharmaceutically acceptable salt thereof. Uses of this substance known in the art particularly relate to its intravenous administration for treatment of heart failure. EP2581082 discloses the use of enoximone in the treatment of status asthmaticus wherein enoximone is used to relieve bronchoconstriction. Beute et al. disclose use of enoximone in allergic airway inflammation (DOI: 10.1172/jci.insight.94888). WO2015/116249 teaches aroylimidazolones for use in treating allergic conditions.

Further aroylimidazolones are known, for example from Schnedler et al. (J Med Chem. 1986 May;29(5):860-2) and from BE883856 and US4405635.

### Summary of the invention

The inventors have surprisingly found that aroylimidazolones advantageously treat the conditions caused by processionary caterpillars. In a first aspect the invention provides an aroylimidazolone or a pharmaceutically acceptable salt thereof for use in the treatment of a condition resulting from exposure to urticating hairs of a caterpillar of the genus *Thaumetopoea.* Preferably the aroylimidazolone is enoximone. The caterpillar of the genus *Thaumetopoea* is preferably selected from the group consisting of *Thaumetopoea processionea* (oak processionary), *Thaumetopoea pityocampa* (pine processionary), *Thaumetopoea bonjeani* (cedar processionary), *Thaumetopoea herculeana, Thaumetopoea pinivora* (eastern pine processionary), *Thaumetopoea solitaria* (pistachio processionary), *Thaumetopoea wilkinsoni,* and *Traumatocampa wilkinsoni,* preferably selected from the group consisting of *Thaumetopoea processionea, Thaumetopoea pinivora,* and *Thaumetopoea pityocampa.* Most preferably it is of the genus *Thaumetopoea processionea.*

The condition resulting from exposure to urticating hairs is preferably selected from the group consisting of cutaneous pathology; dermatitis such as irritant contact dermatitis or pododermatitis; ptyalism; glossitis; conjunctivitis; necrosis such as tongue, lips, or nose necrosis; angioedema; respiratory impairment; rhinitis; stomatitis; edema such as face edema; erucism; skin inflammation; osteomyelitis such as pediatric osteomyelitis; blindness such as panuveitis; ophthalmia such as ophthalmia nodosa; skin irritation due to penetration by urticating hairs; allergic reaction; anaphylactic shock; urticaria; hives; rash such as red rash; thaumetopoein poisoning; and/or itching or pruritis. Preferably it is selected from the group consisting of irritant contact dermatitis; ptyalism; glossitis; conjunctivitis; necrosis; angioedema; rhinitis; stomatitis; edema; erucism; osteomyelitis; blindness; ophthalmia; urticaria; rash; thaumetopoein poisoning; and/or itching. Most preferably it is thaumetopoein poisoning. It is preferred that the condition resulting from exposure is mediated by thaumetopoein.

Within this aspect, the aroylimidazolone is preferably administrated no more often than once per day, or preferably once every four days, or preferably no more often than six, five, four, three, or two times per week, or more preferably no more often than weekly or less often than once per week or each two weeks or each three weeks or monthly or even less frequently. Preferably an improvement of a parameter or a symptom associated with said condition is not seen within 15 seconds or within 1 minute after such compound has been administrated or after the single dose of such compound has been administrated or after the first dose of such compound has been administrated and/or is seen within at least 7 hours after such compound has been administrated or after the single dose of such compound has been administrated or after the first dose of such compound has been administrated, but preferably not within 15 seconds or within 1 minute and/or c. has a duration of or at least 1 day after such compound has been administrated or after the single dose of such compound has been administrated or after the first dose of such compound has been administrated.

In another aspect the invention provides a composition comprising aroylimidazolone for use in the treatment of a condition resulting from exposure to urticating hairs of a caterpillar of the genus *Thaumetopoea.* Preferably the composition is formulated for administration in any one of the following forms: in oral form, in inhalation form, in topical form, in solid form, in fluid form, in cream form, as a transdermal patch, as an injectable preparation, in the form of a sublingual preparation, in the form of a gum or chewing gum, or in the form of a soap, cream, shower gel, or shampoo.

In another aspect the invention provides a kit of parts comprising a composition comprising aroylimidazolone, and further comprising a device or a composition suitable for use during outdoor recreation such as insect repellent, sunscreen, or a tick removal tool.

In another aspect the invention provides a method of treatment of a condition resulting from exposure to urticating hairs of a caterpillar of the genus *Thaumetopoea,* wherein the method comprises administering an aroylimidazolone or a pharmaceutically acceptable salt thereof to a subject exposed to said urticating hairs.

### Description of the invention

The invention provides an aroylimidazolone or a pharmaceutically acceptable salt thereof for use in the treatment of a condition resulting from exposure to urticating hairs of a caterpillar of the genus Thaumetopoea.

### Aroylimidazolone

The invention describes a new use of aroylimidazolone such as aroyl-2H-imidazol-2-on, preferably enoximone or a pharmaceutically acceptable salt thereof. Enoximone is a compound that belongs to this group that can be referred to as aroyl-2H-imidazol-2-on. In particular, the invention involves the use of the compound 1,3-dihydro-4-methyl-5-[4-(methylthio)benzoyl]-2H-imidazol-2-on, also referred to as 4-methyl-5-{[4-methylsulfanyl)phenyl]carbonyl}-2,3-dihydro-1H-imidazol-2-on, or a pharmaceutically acceptable salt thereof. Aroylimidazolones of the invention are preferably 4-aroyl-2H-imidazol-2-ones. Examples of aroyl-2H-imidazol-2-ones are 4-benzoyl-1,3-dihydro-2H-imidazol-2-one; 1,3-dihydro-4-(4-nitrobenzoyl)-2H-imidazol-2-one; 4-benzoyl-1,3-diacetyl-1,3-dihydro-2H-imidazol-2-one; 4-benzoyl-1,3-dihydro-5-(lower alkyl)-2H-imidazol-2-one; 4-benzozyl-1,3-diacetyl-1,3-dihydro-5-methyl-2H-imidazol-2-one; 1,3-dihydro-4-(hydroxybenzoyl)-2H-imidazol-2-one; 1,3-dihydro-4-(hydroxybenzoyl)-5-(lower alkyl)-2H-imidazol-2-one; 1,3-diacetyl-1,3-dihydro-4-(3,4-dimethylbenzoyl)-2H-imidazol-2-one; 1,3-dihydro-4-(3,4-dihydroxybenzoyl)-2H-imidazol-2-one; 1,3-dihydro-4-methyl-5-(4-nitrobenzoyl)-2H-imidazol-2-one, 4-(3-aminobenzoyl)-1,3-dihydro-2H-imidazol-2-one, 4-(4-aminobenzoyl)-1,3-dihydro-2H-imidazol-2-one, and 4-(4-aminobenzoyl)-1,3-dihydro-5-methyl-2H-imidazol-2-one Hereinafter, the terms "enoximone" and "aroyl-2H-imidazol-2-on" will mainly be used, whereby all specific compounds mentioned above are also referred to. A preferred aroylimidazolone is a aroyl-2H-imidazol-2-on or a pharmaceutically acceptable salt thereof, even more preferably it is enoximone or a pharmaceutically acceptable salt thereof.

### Thaumetopoea caterpillars

In preferred embodiments the caterpillar of the genus *Thaumetopoea* is selected from the group consisting of *Thaumetopoea processionea* (oak processionary), *Thaumetopoea pityocampa* (pine processionary), *Thaumetopoea bonjeani* (cedar processionary), *Thaumetopoea herculeana, Thaumetopoea pinivora* (eastern pine processionary), *Thaumetopoea solitaria* (pistachio processionary), *Thaumetopoea wilkinsoni,* and *Traumatocampa wilkinsoni.* More preferably it is selected from the group consisting of *Thaumetopoea processionea, Thaumetopoea pinivora,* and *Thaumetopoea pityocampa;* even more preferably from the group consisting of *Thaumetopoea processionea* and *Thaumetopoea pityocampa.*

In highly preferred embodiments the caterpillar of the genus *Thaumetopoea* is *Thaumetopoea processionea.* In highly preferred embodiments the caterpillar of the genus *Thaumetopoea* is *Thaumetopoea pinivora.* In highly preferred embodiments the caterpillar of the genus *Thaumetopoea* is *Thaumetopoea pityocampa.* A preferred *Thaumetopoea pityocampa* is *Thaumetopoea pityocampa* Schiff.

### Condition to be treated

The conditions caused by processionary caterpillars are generally caused by their urticating hairs also known as urticating bristles, irritating hairs, or burning hairs. The hairs can be inhaled, and they can come into contact with the skin. Animals such as dogs can ingest the hairs, sometimes leading to necrosis of the tongue.

The invention provides the aroylimidazolone for use as described above, wherein the condition resulting from exposure to urticating hairs is selected from the group consisting of cutaneous pathology; dermatitis such as irritant contact dermatitis or pododermatitis; ptyalism; glossitis; conjunctivitis; necrosis such as tongue, lips, or nose necrosis; angioedema; respiratory impairment; rhinitis; stomatitis; edema such as face edema; erucism; skin inflammation; osteomyelitis such as pediatric osteomyelitis; blindness such as panuveitis; ophthalmia such as ophthalmia nodosa; skin irritation due to penetration by urticating hairs; allergic reaction; anaphylactic shock; urticaria; hives; rash such as red rash; thaumetopoein poisoning; and/or itching or pruritis.

Preferably the condition resulting from exposure to urticating hairs is selected from the group consisting of irritant contact dermatitis; ptyalism; glossitis; conjunctivitis; necrosis; angioedema; rhinitis; stomatitis; edema; erucism; osteomyelitis; blindness; ophthalmia; urticaria; rash; thaumetopoein poisoning; and/or itching. More preferably the condition resulting from exposure is mediated by thaumetopoein.

In preferred embodiments the conditions is epidemic caterpillar dermatitis.

The invention involves in particular the use of the above-mentioned compound for the treatment of epidemic caterpillar dermatitis (lepidopterism) caused by caterpillars of the genus *Thaumetopoea,* in particular *Thaumetopoea processionea* and *Thaumetopoea pityocampa,* manifested as, but not restricted to, a papular rash, pruritus, dizziness, conjunctivitis, fever, and, if inhaled pharyngitis, gagging, vomiting and respiratory distress, including asthma, anaphylaxis or even death. This is a non-exhaustive list of symptoms caused by the bristles (setae) of caterpillars of the genus *Thaumetopoea,* in particular *Thaumetopoea processionea* and *Thaumetopoea pityocampa,* in particular by the urticating toxin in those setae, the protein thaumetopoein, which triggers the adverse reactions as mentioned above, all of which can be treated effectively using the present invention. Surprisingly, aroylimidazolines described in this invention has proven to reduce or even eliminate altogether the distress associated with the above-mentioned symptoms.

The use of aroyl-2H-imidazol-2-on, preferably enoximone or any pharmaceutically acceptable salt of either leads to a marked improvement of the quality of life of the patient involved. An additional effect of the use of aroyl-2H-imidazol-2-on, preferably enoximone or a pharmaceutically acceptable salt thereof in accordance with the invention is that it relieves symptoms more adequately than antihistamine based therapies. In preferred embodiments is provided the aroylimidazolone for use as described above, wherein the condition resulting from exposure to urticating hairs is not an allergic reaction, preferably wherein the aroylimidazolone is not for achieving bronchodilation.

Epidemic caterpillar dermatitis as caused by caterpillars of the genus *Thaumetopoea,* is a relatively new disorder, formerly found solely in warmer climates, but now experiencing an expansion range towards higher latitudes and altitudes due to the current climate warming. The symptoms are hard to treat, as regular anti-histamines appear to provide little relieve. The symptoms are caused by the bristles (setae) on the backs of older caterpillars which are covered with up to 63,000 pointed defensive bristles, sized between 0.2 and 0.3 millimeters, and which contain an urticating toxin, the protein thaumetopoein. The setae break off readily and transmission of the hairs can be airborne, by ground contact, via plants or grass, or even by water contact in still water e.g. garden ponds. Also mowing a lawn can bring a person into contact with these hairs. The toxicity of the hairs remains active beyond the normal life cycle of the caterpillar and in some cases can remain a problem for several seasons.

When sensitive to the protein, symptoms including a papular rash, pruritus, dizziness, conjunctivitis, fever, and, if inhaled, pharyngitis, gagging, vomiting and respiratory distress, including asthma, anaphylaxis or even death can occur. Mammals are generally sensitive to the protein. In addition to humans, cats, dogs and other mammal pets are equally affected, with symptoms including rashes, gagging and vomiting, respiratory distress, inflammation of the mouth, swelling of the tongue, conjunctivitis, hyper-salivation and death.

The symptoms, problems or consequences of caterpillar dermatitis can be manifold, ranging from light irritations of the skin and mucosa, as well as to a deterioration in condition, or even to lasting invalidity and death, due to the secondary effects, mainly including respiratory complications. In particular skin disorders are often highly socially charged, which strongly affects the patient's quality of life. The diagnostic criteria for skin disorders caused by caterpillars of the genus *Thaumetopoea* are determined by contact and can manifest themselves as itchiness, redness, and blistering; respiratory distress can manifest itself as irritation and inflammation of the mucosa in mouth and throat, coughing, asthma attacks, status asthmaticus and choking. Skin and RAST tests can be indicative in this regard. In the case of disorders caused by caterpillars of the genus *Thaumetopoea,* the most important diagnostic criteria can be the symptoms as described by the patients themselves.

### Previous methods of treatment

Disorders caused by caterpillars of the genus *Thaumetopoea* are usually treated by antihistamines, menthol powder as an antipruritic to reduce itching, and lukewarm baths. Treatment usually involves a combination of the above remedies. In case of respiratory complications oxygen by means of an oxygen device might be necessary, and in case of secondary complications such as asthma and anaphylaxis, treatment for these specific disorders will have to be involved.

In general, it can be observed that a high percentage of patients react insufficiently or inadequately to the traditional treatments as described above. Increasing the dose of traditional medication usually does not lead to a reduction of symptoms. Non-treatment is possible, but not the preferred option: in serious cases that could lead to severe disability, especially due to a decline of the patient's condition, excoriation of the skin, and (extreme) shortness of breath. In this regard it is needless to say that the patient should try to avoid or avert the triggering bristles as much as possible.

It is therefore clear that the treatment of caterpillar dermatitis as caused by caterpillars of the genus *Thaumetopoea* is a medical challenge. The group of patients is large and varied, and presents many possible options, ranging from mere light maintenance treatments to long and exhausting treatments because of complications.

### Treatment

In the context of the invention, treating, curing, and/or stabilizing the disease or condition associated with caterpillar dermatitis as caused by caterpillars of the genus *Thaumetopoea* may mean that:
- at least a symptom of this disease or condition has been improved,
- at least a parameter associated with this disease or condition has been improved.
Depending on the identity of the disease or condition, a symptom may be at least one of the following symptoms:
- itching of the skin, red skin and urticarial
- sneezing, coughing, nasal blockage, nasal discharge, itching of the nose, itching of the eye and itching and/or inflammation of the oral mucosa
- wheezing, chest tightness, dyspnoea

The improvement may be measured and/or visible (i.e. significant improvement) after one hour of the onset of the treatment, or 2, 3, 4, 5, 6, 7, 8, 9, 10, 11, 12, 13, 14, 15, 16, 17, 18, 19, 20 ,21, 22, 23, 24 hours, or after one day, two days, four days, or after at least one week, one month, six months after the onset of the treatment or more.

It is encompassed by the invention that the treatment may consist of the administration of a single dose, or two doses or three doses of the compound. It is also encompassed by the invention, that the treatment may comprise or consist of an administration frequency of no more often than once per day, or preferably once every four days, or preferably no more often than six, five, four, three, or two times per week, or more preferably no more often than weekly or less often than once per week or each two weeks or each three weeks or monthly or even less frequently.

During the course of the treatment, the symptom may also occur less and less frequently. As a result a subject may adapt the frequency of administration of enoximone depending on the severity of occurrence of a symptom. Enoximone may be administrated once or twice a week as a first dose and depending on the evolution of the frequency of the occurrence of a symptom, it may subsequently be administrated every week, subsequently every two weeks. A symptom may be assessed by interview or anamnesis, or alternately by known tests or appropriate parameters.

In a preferred embodiment, the present invention provides a new indication or a new medical use for aroyl-2H-imidazol-2-on, preferably for the substance enoximone, which can be used for caterpillar dermatitis as caused by caterpillars of the genus Thaumetopoea, with direct as well as long term maintenance results. Enoximone can effectively counteract the adverse symptoms of caterpillar dermatitis as caused by caterpillars of the genus *Thaumetopoea.* Preferably, in such a case a long-lasting effect is achieved. Related to the description above, an embodiment of the invention is the use of aroyl-2H-imidazol-2-on, preferably enoximone, for ameliorating and/or treating a disease or condition in a subject, wherein said disease or condition is caterpillar dermatitis as caused by caterpillars of the genus Thaumetopoea.

### Form of the substance

According to the present invention, aroyl-2H-imidazol-2-on, preferably enoximone is a solid substance which can for example be processed into tablets, suppositories, enemas, vaginal tablets, suspensions, powders, (transdermal) patches and creams. In the above forms, the patients can manage the intake or application themselves, obviously in accordance with the prescriptions of the treating physician. An injectable preparation (intravenous, subcutaneous, intracutaneous or intramuscular), for example a depot preparation with delayed release, is another possible form. This can be administered in an outpatient centre or by a GP or physician.

### Administration and quantity

According to the present invention, the active substance aroyl-2H-imidazol-2-on, preferably Enoximone, can be taken both as a fluid and as a solid and can also be applied to the skin. The fluid oral form of administration is a suspension. The possible solid oral forms are dispersible tablets, effervescent tablets, coated tablets (capsules or granules), delayed release tablets or modified release tablets (capsules or granules) (slow release), liquid soft gel capsules, gums or chewing gums, sublingual preparations, capsules, powders, granules, patches, enemas, tablets, preferably vaginal tablets, suppositories or creams or ointment, oral solutions, oral suspensions. Preferred tablet or capsule formulations are provided in the examples. An injectable depot preparation (sub/intracutaneous or intramuscular) is also conceivable. For the cream version, enoximone can be added to a dermatological medium in a solid or fluid form, possibly containing further excipients. Sublingual preparation is attractive as it exhibits a quick resorption (i.e. less than 60 s) and it is unobstructive. Gum or chewing preparation is attractive especially for children as it is not perceived as a drug or medicament. It is also not obstructive. Enoximone may also be formulated to be administrated as a soap, cream, shower gel/cream and/or shampoo (topical use). This is especially attractive for skin disorder patients. They often are very tender to normal soaps and shampoos.

When enoximone is administered orally, it is preferable that the quantity of active substance is situated in a range from 0.01 to 2 mg per kg body weight, preferably 0.01 to 0.8 mg/kg, preferably 0.01 to 8 mg/kg, preferably at least 0.05 mg/kg, more preferably at least 0.1 mg/kg, even more preferably at least 0.2 mg/kg and most preferably at least 0.25 mg/kg.

Furthermore, the quantity is preferably at most 1.5 mg/kg, more preferably at most 1 mg/kg, even more preferably at most 0.8 mg/kg and most preferably at most 0.5 mg/kg. The maximum daily dose of the active substance is preferably 2 mg/kg of body weight yet at most 250 mg/kg body weight, more preferably at most 200 mg/kg body weight, even more preferably at most 150 mg/kg body weight.

The solid form of administration can for example be made available in dosage units
of 5, 10 and 20 mg, relative to the quantity of aroyl-2H-imidazol-2-on, preferably enoximone.

When aroyl-2H-imidazol-2-on, preferably enoximone, is administered in inhalation form, it is preferable that the effective release of the active substance is situated in a range from 0.01 to 5 15 mg, preferably from 0.01 to 8 mg preferably at least 0.05 mg, more preferably at least 0.1 mg, even more preferably at least 0.2 mg and most preferably at least
0.3 mg, for example 0.5 mg. Furthermore, the quantity is preferably at most 15 mg, more preferably at most 10 mg, even more preferably at most 8 mg and most preferably at most
5 mg.

The substance can be made available as a suspension, for example in 5, 10, 20, 50 or
100 ml containers. The aroyl-2H-imidazol-2-on, preferably enoximone, content can for example be 1 mg/ml.

When aroyl-2H-imidazol-2-on, preferably enoximone is administered in cream form, it is preferable that the effective concentration (w/w) of aroyl-2H-imidazol-2-on, preferably

15 enoximone in the cream is situated in a range from 0.01 to 10%, preferably at least 0.05%, more preferably at least 0.8%, and preferably at most 8%, more preferably at most 5%, even more preferably at most 3%.

Enoximone may also be formulated to be administrated as a soap, cream, shower gel/cream and/or shampoo in dosage forms of 5, 10, 15, 20, 25, 50, or 100 mg per dose.

In an embodiment of the invention, the use of an aroyl-2H-imidazol-2-on, preferably enoximone or a pharmaceutically acceptable salt thereof is its use for the preparation of a medicament for ameliorating and/or treating a disease or condition in a subject, wherein said disease or condition is preferably caterpillar dermatitis as caused by caterpillars of the genus Thaumetopoea. Each feature of this use has been already defined herein.

In an embodiment of the invention, a pharmaceutical composition comprising aroyl-2H-imidazol-2-on, preferably enoximone or a pharmaceutically acceptable salt thereof in an active effective quantity, preferably in a dosage unit of 5, 10 or 20 mg, based on the quantity of the active ingredient, is provided. In said pharmaceutical composition, said aroyl-2Himidazol-2-on can be enoximone or a pharmaceutically acceptable salt thereof. Each feature of this pharmaceutical composition has been already defined herein.

The dosage and frequency of the above-mentioned forms can be adjusted according to the body weight, the patient's experiences, and findings from future tests and experiments.

Initial dosages can be estimated from in vivo data, e.g., animal models, using techniques that are well known in the art. One having ordinary skill in the art could readily optimize administration to humans based on animal data. The amount of molecules administered will, of course, be dependent on the subject being treated, on the subject's weight, the severity of the affliction, the manner of administration and the judgment of the prescribing physician. The moment of administration can be based on the moment of the occurrence of symptoms, for example according to the patient's experiences with regard to the disorder. The moment of administration can be based on a dosage regime that can comprise daily administrations, or less frequent administrations such as once every four days, twice a week, or weekly or each two weeks or each three week or monthly or even less frequently. A relatively less frequent administration (i. e. once every four days, twice a week, or weekly or each two weeks or each three week or monthly or even less frequently) is attractive since possible toxicity or side effect is minimized and patient's freedom and self-image are optimized.

In addition such less frequent administration allows a de-escalation regime: the patient himself may decrease the frequency of administration based on his own perceived symptoms.

Aroyl-2H-imidazol-2-on, preferably enoximone, can be administered or taken in an appropriate manner in daily, multi-daily, weekly or multi-weekly doses.

### Formulations

According to the present invention, aroylimidazolone such as aroyl-2H-imidazol-2-on, preferably enoximone is a solid substance which can for example be processed into tablets, suppositories, enemas, vaginal tablets, suspensions, powders, (transdermal) patches and creams. In the above forms, the patients can manage the intake or application themselves, obviously in accordance with the prescriptions of the treating physician. An injectable preparation (subcutaneous, intracutaneous or intramuscular), for example a depot preparation with delayed release, is another possible form. This can be administered in an outpatient centre or by a GP or physician. Unlike the existing form of administration consisting of a fluid, dissolved through a special procedure (corresponding use in heart failure), which must always be administered in a clinical situation, these forms of application are new and much more accessible.

In an embodiment, aroyl-2H-imidazol-2-on, preferably enoximone is not injected intravenously, more preferably not injected via a bolus injection.

Aroyl-2H-imidazol-2-on, preferably enoximone can be administered or taken in an appropriate manner in daily, multi-daily, weekly or multi-weekly doses.

In an embodiment, aroyl-2H-imidazol-2-on, preferably enoximone is for use as defined herein wherein the subjects show limited or no response to treatment with at least one of the following: beta-2 agonists, parasympatholytics, anticholinergics, aminophyllines, antihistamines, magnesium sulphate, corticosteroids, and cytostatics, preferably with antihistamines.

The inventors have found that enoximone is extremely suitable for the above purposes. Intravenous administration lead to improvements within as little as one minute, and within ten minutes if administered orally. As maintenance medication, it ensures lasting (from a few days to several weeks or several months) improvement of the symptoms.

The inventors have found that treatment of all of the above conditions is surprisingly adequate, and above all, immediate. Surprisingly efficient results were obtained when aroyl-2H-imidazol-2-on, preferably enoximone was administered at low doses and/or at a low frequency. Low doses in this context means 10 mg a day or less than 10 mg per day, or 0.01 to 8 mg per day or less. In preferred embodiments, not more than 10 mg is administered per 3 days. Potential side effects may be circumvented through the use of such low doses. In the context of the invention, low frequency means the present invention allows for dosage regimes that involve an intake schedule featuring intake moments that occur daily, once every four days, weekly, twice a week, preferably six, five, four, or three times a week, more preferably even less often (once per week, once per month, every two, three, four month or even less often), thus relieving the burden on the patient. Treatment with enoximone and the subsequent decrease of complaints and recovery of condition, will improve the quality of life of many patients considerably.

### Kit of parts

The invention provides a kit of parts comprising aroylimidazolone, preferably enoximone, and further comprising a device or composition suitable for use during outdoor recreation such as insect repellent, sunscreen, or a tick removal tool. Other further comprised compositions can be mosquito repellant such as DEET, a disinfectant, oral rehydration salts, and bear mace. Other objects can be mosquito nets, utility knives, nets, lighters, and tents.

### DEFINITIONS

In this document and in its claims, the verb "to treat" is used preferably to refer to delaying, ameliorating, stabilizing, preventing, or curing a disease or condition, or the progress of a disease or condition, in a subject. More preferably, the disease is ameliorated, stabilized, or cured, more preferably it is ameliorated. Treatment preferably comprises administration of an effective dose of the aroylimidazolone.

A subject to be treated is preferably a subject in need of treatment. Preferably, a subject is a warm-blooded subject, more preferably a mammal. Preferred mammals are humans and domesticated mammals. Preferred domesticated mammals are dogs or horses. In preferred embodiments, a subject is a human or a dog. In preferred embodiments, a subject is a dog. In preferred embodiments, a subject is a human. In preferred embodiments, a subject is not a human.

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a product, an assay device respectively a method or a use as defined herein may comprise additional component(s) respectively additional step(s) than the ones specifically identified, said additional component(s), respectively step(s) not altering the unique characteristic of the invention.

In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

### Examples

The invention is explained in more detail below with a number of tests and examples, which are not to be construed as limiting the scope of the invention. The invention is not limited to the forms of implementation described in the cases given as examples. The invention also extends to each combination of measures as described above, independently from each other.

### Example 1 - Trials

### Trial 1

A 65 year old man presented himself with an extreme form of caterpillar dermatitis as caused by caterpillars of the genus *Thaumetopoea,* in this case *Thaumetopoea processionea.* He had been bothered by the affliction for several weeks and complained of severe itching, of redness of the skin, and of inflammation of the skin. This lead to loss of sleep, loss of concentration, and scratching to the point of skin abrasion.

Traditional treatment, advised by his GP, had not led to alleviation of the symptoms.

The subject was administered enoximone. The dose administered was 10 mg, taken orally in a drink of water. Within 10 minutes the itching subsided, and within a day all signs of redness were gone. Three days after the administration the symptoms had not returned.

### Example 2 - formulations of aroylimidazolones

Non-limiting examples of formulations are provided. In each of the examples below, each of the specific substances used in combination with enoximone may have been replaced by another equivalent substance having the same function as indicated in the table (i.e other filler, binder, disintegrant, lubricant, glidant, sweetener, flavor, viscosity modifier, surfactant, tonicity agent, complexing agent, preservative and/or carrier).

### 2.1 Immediate Release (IR) tablets or capsules

The active drug substance can be in crystalline or amorphous form.

### 2.1.1 Direct compression IR tablet

Direct compression formulations are prepared by dry blending of components.

| **Component** | **mg** | **Action** |
|---|---|---|
| Enoximone | 10 | Active drug substance |
| Lactose | 50 | Filler |
| Microcrystalline cellulose | 30 | Filler/binder |
| Croscarmellose sodium | 4.5 | Disintegrant |
| Magnesium stearate | 0.5 | Lubricant |
| **Total** | 100 | |

### 2.1.2 Direct compression IR tablet

| **Component** | **mg** | **Action** |
|---|---|---|
| Enoximone | 10 | Active drug substance |
| Lactose | 75 | Filler |
| Povidone | 10 | Binder |
| Crospovidone | 4.5 | Disintegrant |
| Magnesium stearate | 0.5 | Lubricant |
| **Total** | 100 | |

### 2.1.3 Wet granulation IR tablet

Wet granulation involves mixing of excipients and drug substance with a binder solution using granulation equipment. Addition of extra-granular excipients is also part of the process; the formula below shows the overall composition without differentiating intra and extragranular components.

| **Component** | mg | **Action** |
|---|---|---|
| Enoximone | 10 | Active drug substance |
| Microcrystalline cellulose | 158 | Filler |
| Povidone | 10 | Binder |
| Sodium starch glycolate | 21 | Disintegrant |
| Magnesium stearate | 1 | Lubricant |
| **Total** | 200 | |

### 2.1.4 capsule composition

Granules manufactured as above and filled into capsules (e.g. hard gelatin).

| **Component** | mg | **Action** |
|---|---|---|
| Enoximone | 20 | Active drug substance |
| Microcrystalline cellulose | 108 | Filler |
| Povidone | 6 | Binder |
| Croscarmellose sodium | 15 | Disintegrant |
| Hydrophobic silica | 1 | Glidant |
| **Total** | 150 | |

### 2.2 Orodispersible tablets (ODT)

The active drug substance can be in crystalline or amorphous form. Manufactured as direct compression.

| **Component** | mg | **Action** |
|---|---|---|
| Enoximone | 10 | Active drug substance |
| Mannitol | 82.6 | Filler |
| Crospovidone | 5 | Binder |
| Acesulfame K | 0.5 | Sweetener |
| Hydrophobic silica | 0.5 | Glidant |
| Magnesium stearate | 1 | Lubricant |
| Orange flavour | 0.3 | Flavour |
| Peppermint flavour | 0.1 | Flavour |
| **Total** | 100 | |

### 2.3 Aqueous suspension for nasal inhalation

The active drug substance is micronised. Aqueous suspension buffered at pH 4.5:

| **Component** | % (w/w) | **Action** |
|---|---|---|
| Enoximone | 0.06 | Active drug substance |
| Croscarmellose sodium | 0.15 | Viscosity modifier |
| Polysorbate 80 | 0.05 | Surfactant |
| Sodium chloride | 0.9 | Tonicity agent |
| Disodium edetate | 0.05 | Complexing agent |
| Benzalkonium chloride | 0.02 | Preservative |
| Water for injection | q.s. | Carrier |

It can be concluded based on the tests described above that treatment with aroylimidazolone such as enoximone, possibly in addition to the traditional treatment, is a very adequate and advantageous approach for subjects exposed to *Thaumetopoea.* Other expensive treatments can possibly be omitted. In addition to the fact that quality of life can be improved considerably for a subject thanks to treatment with aroylimidazolones, this treatment offers substantial cost savings to society and can mitigate the unhinging effect of *Thaumetopoea* plagues.

## Claims

1. An aroylimidazolone or a pharmaceutically acceptable salt thereof for use in the treatment of a condition resulting from exposure to urticating hairs of a caterpillar of the genus *Thaumetopoea.*

2. The aroylimidazolone for use according to claim 1, wherein the caterpillar of the genus *Thaumetopoea* is selected from the group consisting of *Thaumetopoea processionea* (oak processionary), *Thaumetopoea pityocampa* (pine processionary), *Thaumetopoea bonjeani* (cedar processionary), *Thaumetopoea herculeana, Thaumetopoea pinivora* (eastern pine processionary), *Thaumetopoea solitaria* (pistachio processionary), *Thaumetopoea wilkinsoni,* and *Traumatocampa wilkinsoni,* preferably selected from the group consisting of *Thaumetopoea processionea, Thaumetopoea pinivora,* and *Thaumetopoea pityocampa.*

3. The aroylimidazolone for use according to claim 1 or 2, wherein the caterpillar is of the genus *Thaumetopoea processionea.*

4. The aroylimidazolone for use according to any one of claims 1-3, wherein the aroylimidazolone is enoximone.

5. The aroylimidazolone for use according to any one of claims 1-4, wherein the condition resulting from exposure to urticating hairs is selected from the group consisting of cutaneous pathology; dermatitis such as irritant contact dermatitis or pododermatitis; ptyalism; glossitis; conjunctivitis; necrosis such as tongue, lips, or nose necrosis; angioedema; respiratory impairment; rhinitis; stomatitis; edema such as face edema; erucism; skin inflammation; osteomyelitis such as pediatric osteomyelitis; blindness such as panuveitis; ophthalmia such as ophthalmia nodosa; skin irritation due to penetration by urticating hairs; allergic reaction; anaphylactic shock; urticaria; hives; rash such as red rash; thaumetopoein poisoning; and/or itching or pruritis.

6. The aroylimidazolone for use according to any one of claims 1-5, wherein the condition resulting from exposure to urticating hairs is selected from the group consisting of irritant contact dermatitis; ptyalism; glossitis; conjunctivitis; necrosis; angioedema; rhinitis; stomatitis; edema; erucism; osteomyelitis; blindness; ophthalmia; urticaria; rash; thaumetopoein poisoning; and/or itching.

7. The aroylimidazolone for use according to any one of claims 1-6, wherein the condition resulting from exposure to urticating hairs is not an allergic reaction, preferably wherein the aroylimidazolone is not for achieving bronchodilation.

8. The aroylimidazolone for use according to any one of claims 1-6, wherein the condition resulting from exposure is mediated by thaumetopoein.

9. The aroylimidazolone for use according to any one of claims 1-8, wherein the aroylimidazolone is administrated no more often than once per day, or preferably once every four days, or preferably no more often than six, five, four, three, or two times per week, or more preferably no more often than weekly or less often than once per week or each two weeks or each three weeks or monthly or even less frequently.

10. The aroylimidazolone for use according to any one of claims 1-9, wherein an improvement of a parameter or a symptom associated with said condition
a. is not seen within 15 seconds or within 1 minute after such compound has been administrated or after the single dose of such compound has been administrated or after the first dose of such compound has been administrated and/or
b. is seen within at least 7 hours after such compound has been administrated or after the single dose of such compound has been administrated or after the first dose of such compound has been administrated, but preferably not within 15 seconds or within 1 minute and/or
c. has a duration of or at least 1 day after such compound has been administrated or after the single dose of such compound has been administrated or after the first dose of such compound has been administrated.

11. The aroylimidazolone for use according to any one of claims 1-10, wherein the subject has not been sensitized to urticating hairs of a caterpillar of the genus *Thaumetopoea* prior to exposure to thereto.

12. Composition comprising aroylimidazolone as defined in claims 1 or 4, for use as defined in any one of claims 1-11.

13. The composition according to claim 12, wherein the composition is formulated for administration in any one of the following forms:
i) in oral form, preferably of a quantity of active substance situated in a range from 0.01 to 2 mg/kg of body weight;
ii) in inhalation form, preferably in a quantity whereby the effective release of active substance is situated in a range from 0.01 to 15 mg;
iii) in topical form, preferably in cream form, whereby the effective concentration (w/w) of aroylimidazolone is preferably situated in a range from 0.01 to 10%;
iv) in solid form, preferably as dispersible tablet, effervescent tablet, coated tablet, tablet with delayed release, sublingual preparation, gum or chewing gum, capsule or powder, with an optionally added adjuvant or carrier;
v) in fluid form, wherein an aroylimidazolone is added to a compatible dissolving, suspending or emulsifying medium;
vi) in cream form, wherein an aroylimidazolone is added to a compatible dermatological medium;
vii) as a transdermal patch, preferably in a maximum effective aroylimidazolone dose of 100 mg/day, more preferably as a transdermal patch with delayed release;
viii) as an injectable preparation, preferably subcutaneous or intramuscular, more preferably as a depot preparation;
ix) in the form of a sublingual preparation, preferably in dosage forms of 5, 10, 15, 20, 25, 50, or 100 mg per dose;
x) in the form of a gum or chewing gum, preferably in dosage forms of 5, 10, 15, 20, 25, 50, or 100 mg per dose;
xi) in the form of a soap, cream, shower gel, or shampoo, preferably in dosage forms of 5, 10, 15, 20, 25, 50, or 100 mg per dose.

14. Kit of parts comprising a composition as defined in claim 12 or 13, and further comprising a device or composition suitable for use during outdoor recreation such as insect repellent, sunscreen, or a tick removal tool.

15. Method of treatment of a condition resulting from exposure to urticating hairs of a caterpillar of the genus *Thaumetopoea,* wherein the method comprises administering an aroylimidazolone or a pharmaceutically acceptable salt thereof to a subject exposed to said urticating hairs.
